# NEW EUROPEAN PATENT SPECIFICATION

(11) **EP 2 815 753 B2**
(45) Date of publication and mention of the opposition decision: **05.11.2025**
(45) Mention of the grant of the patent: 16.05.2018
(21) Application number: 13749171.8
(22) Date of filing: 14.02.2013
(51) Int. Cl.: A61K 31/7072, A61K 9/20, A61K 9/28, A61K 31/513, A61K 47/10, A61K 47/26, A61K 47/32, A61K 47/36, A61K 47/38, A61P 35/00, A61P 43/00

(54) **AN ORALLY ADMINISTRABLE PHARMACEUTICAL COMPOSITION COMPRISINGA,A,A-TRIFLUOROTHYMIDINE AND 5-CHLORO-6-(2-IMINOPYRROLIDINE-1-YL)METHYL-2,4 (1H,3H)-PYRIMIDINE DIONE HYDROCHLORIDE**
ORAL VERABREICHBARE PHARMAZEUTISCHE ZUSAMMENSETZUNG, UMFASSENDA,A,A-TRIFLUORTHYMIDIN UND 5-CHLOR-6-(2-IMINOPYRROLIDIN-1-YL)METHYL-2,4(1 H,3H)-PYRIMIDINDION-HYDROCHLORID
COMPOSITION PHARMACEUTIQUE POUVANT ÊTRE ADMINISTRÉE PAR VOIE ORALE COMPRENANT DE L'A,A,A-TRIFLUOROTHYMIDINE ETDU CHLORHYDRATE DE 5-CHLORO-6-(2-IMINOPYRROLIDIN-1-YL)MÉTHYL-2,4(1H,3H)PYRIMIDINEDIONE

(30) Priority: 15.02.2012 JP 2012031143
(43) Date of publication of application: 24.12.2014
(73) Proprietor: Taiho Pharmaceutical Co., Ltd., Chiyoda-ku, Tokyo 101-8444 (JP)
(72) Inventor: OHNISHI, Yoshito, Tokushima-shi, Tokushima 771-0194 (JP)
(74) Representative: Wächtershäuser & Hartz Patentanwaltspartnerschaft mbB
(86) International application number: PCT/JP2013/053513
(87) International publication number: WO 2013/122134

(56) References cited:
- EP-A1- 1 849 470
- EP-A2- 2 005 962
- WO-A1-2006/080327
- WO-A1-96/30346
- JP-A- 2001 131 075
- JP-A- 2005 112 744
- JP-A- 2005 314 413
- JP-A- 2007 284 390
- JP-A- H11 322 604
- US-A1- 2006 167 031
- EMURA TOMOHIRO ET AL: "Potentiation of the antitumor activity of .alpha., .alpha., .alpha.-trifluorothymidine by the co-administration of an inhibitor of thymidine phosphorylase at a suitable molar ratio in vivo", INTERNATIONAL JOURNAL OF ONCOLOGY, DEMETRIOS A. SPANDIDOS ED. & PUB, GR, vol. 27, no. 2, 1 January 2005 (2005-01-01), pages 449 - 455, XP009140485, ISSN: 1019-6439

## Description

### [Technical Field]

The present invention relates to an orally administrable pharmaceutical composition comprising α,α,α-trifluorothymidine (FTD) and 5-chloro-6-(2-iminopyrrolidine-1-yl)methyl-2,4(1H,3H)-pyrimidine dione hydrochloride (TPI).

### [Background Art]

A combination drug comprising α,α,α-trifluorothymidine (FTD) and 5-chloro-6-(2-iminopyrrolidine-1-yl)methyl-2,4(1H,3H)-pyrimidine dione hydrochloride (TPI) is an anti-tumor agent in which FTD, which has an action for inhibiting thymidylate formation and an action for inhibiting DNA synthesis by incorporation into DNA to exert an anti-tumor effect, is combined with TPI, which has an action for inhibiting thymidine phosphorylase, to thereby suppress degradation of FTD in vivo and enhance the anti-tumor effect (Patent Literature 1).

An anti-tumor agent "TAS-102" in which FTD and TPI are combined in a molar ratio of 1:0.5 is now under development as an orally administrable formulation (Non Patent Literatures 1 and 2). As for the orally-administrable TAS-102 formulation, tablets, granules, capsules, and the like are known so far (Patent Literatures 1 and 2). However, the quality, particularly the storage stability of the formulation has not been sufficiently investigated.

In the case of formulation, in order that medicaments are orally administered with ease, excipients, binders, disintegrating agents, lubricants, taste-masking agents, and the like are usually allowed to be contained, in addition to the active ingredient. Of these, excipients are added to increase the bulk to thereby adjust the size and mass of oral medicaments to a size and mass suitable for handling and ingestion. The mass proportion of excipients often becomes large relative to the amount of medicaments. Accordingly, excipients among formulation additives have large influence on the stability of formulations, and have to be chosen with due care.

Meanwhile, in medical settings, in order to prevent accidental ingestion and to enhance medication compliance, one-dose packaging to package various medicaments into each one dosage form is promoted, and thus, stable and high-quality formulations are desired even without moisture-proof packaging. Also, if moisture-proof packaging becomes unnecessary, advantages are brought about, such as elimination of trouble of opening packages and elimination of waste packages.

### [Citation List]

### [Patent Literature]

[Patent Literature 1]
   International Publication No. WO 96/30346
[Patent Literature 2]
   International Publication No. WO 2006/80327

### [Non Patent Literature]

[Non Patent Literature 1]
   International Journal of Oncology 25: 571-578, 2004
[Non Patent Literature 2]
   Invest New Drugs 26(5): 445-54, Oct 2008.

### [Summary of Invention]

### [Technical Problem]

The present inventor has added various formulation additives to the above FTD and TPI, and has investigated the storage stability of the resulting compositions under various conditions. Then, it has been proved that the amount of FTD and TPI related substances were increased when stored particularly under high-humidity conditions depending on types of formulation additives added.

Accordingly, an object of the present invention is to provide an FTD and TPI-containing orally administrable pharmaceutical composition which can be orally administered and whose active ingredients are stable even under high-humidity conditions.

### [Solution to Problem]

Thus, the present inventor has added various additives to FTD and TPI and evaluated the storage stability, and has found that a stable orally administrable pharmaceutical composition in which mass of related substances is not substantially increased even stored in the case of using a sugar having a high critical relative humidity, completing the present invention.

That is, the present invention provides an orally administrable pharmaceutical composition as defined by claim 1.

Also, the present invention provides an orally administrable pharmaceutical formulation comprising the above-described orally administrable pharmaceutical composition, which is coated.

### [Advantageous Effects of Invention]

According to the present invention, high-quality formulations having secured formulation stability even under high-humidity conditions can be provided to patients and medical staffs.

### [Description of Embodiments]

The active ingredients of the orally administrable pharmaceutical composition of the present invention are FTD and TPI. The molar ratio of FTD and TPI contained in the composition is 1:0.5. Also, the content of FTD per dosage unit of the orally administrable pharmaceutical composition is preferably from 5 to 35 mg and more preferably from 15 to 20 mg.

Although the contents of FTD and TPI, which are the active ingredients of the oral pharmaceutical composition of the present invention, depend on formulation forms and regimens, and may be selected without particular limitation and as appropriate, the amount of each active ingredient in pharmaceutical composition is preferably from of the order of 1 to 40% by mass.

The orally administrable pharmaceutical composition of the present invention, to which sugars having a critical relative humidity of 85% or more at 25°C as an excipient is added, suppresses increases in FTD and TPI related substances even stored under high-humidity conditions. A "critical relative humidity" herein means a well-known indicator representing the hygroscopicity, and refers to a relative humidity when a rapid increase in the amount of moisture absorbed in a sample is observed in the case where the relative humidity is increased. The critical relative humidity can be checked by measuring the change in the weight of a sample at 25°C and a relative humidity of from 10 to 95% using, for example, a moisture sorption analyzer (DVS-1, Surface Measurement Systems Ltd.). "A critical relative humidity at 25°C is 85% or more" means that moisture is not substantially absorbed when the relative humidity at 25°C is less than 85%. Also, "no critical relative humidity" means that moisture is absorbed at a low humidity depending on the humidity, and a rapid increase in the amount of moisture absorbed associated with an increase in the relative humidity is not observed.

The sugar having a critical relative humidity of 85% or more at 25°C in the oral pharmaceutical composition of the present invention is selected from lactose (including anhydride and hydrate), sucrose, mannitol, and erythritol, and lactose, sucrose, or mannitol is preferred, and lactose or mannitol is particularly preferred. It should be noted that these sugars may be used singly or in combination of two or more.

Of these sugars, from a viewpoint of the stability of the aforementioned FTD and TPI, disaccharides or sugar alcohols having a critical relative humidity of 85% or more at 25°C are preferred, disaccharides or sugar alcohols having a critical relative humidity of 90% or more at 25°C are more preferred, and disaccharides or sugar alcohols having a critical relative humidity of 95% or more at 25°C are particularly preferred. Specifically, lactose (including anhydride and hydrate), sucrose, mannitol, trehalose, maltose, maltitol, or erythritol is preferred, lactose, sucrose, mannitol, trehalose, or maltose is more preferred, lactose, sucrose, or mannitol is more preferred, and lactose or mannitol is particularly preferred. It should be noted that these sugars may be used singly or in combination of two or more.

The content of the sugar having a critical relative humidity of 85% or more in the orally administrable pharmaceutical composition of the present invention is, from viewpoints of the stability of FTD and TPI and of the function as an excipient, 3.6 parts by mass or more, preferably from 3.6 to 50 parts by mass, more preferably from 3.7 to 25 parts by mass, and particularly preferably from 3.7 to 10 parts by mass, based on 1 part by mass of FTD.

Also, disintegrating agents are added to the orally administrable pharmaceutical composition of the present invention in order to secure good disintegrability at oral administration. However, most disintegrating agents have no critical relative humidity, and may impair the stability of FTD and TPI depending on the types. The disintegrating agent in the orally administrable pharmaceutical composition of the present invention is partly pregelatinized starch. The content of the disintegrating agent is, from a viewpoint of combining the stability of FTD and TPI in the pharmaceutical composition of the present invention and the disintegrability of the pharmaceutical composition from 3 to 7% by mass in the total amount of the pharmaceutical composition.

Although the contents of FTD and TPI, which are the active ingredients of the orally administrable pharmaceutical composition of the present invention, depend on formulation forms and regimens, and may be selected without particular limitation and as appropriate, the amount of each active ingredient in the total amount of the pharmaceutical composition is preferably from of the order of 1 to 40% by mass. Of additives for the pharmaceutical composition, the proportion of the sugar having a critical relative humidity of 85% or more at 25°C in the present invention is, from a viewpoint of the stability of the active ingredients, preferably from 50 to 100% by mass, more preferably a range from 70 to 100% by mass, and particularly preferably from 70 to 98% by mass, in the total amount of the additives.

Alternatively, excipients other than the sugar having a critical relative humidity of 85% or more at 25°C may be added to the orally administrable pharmaceutical composition of the present invention. From a viewpoint of the stability of the active ingredients, the proportion of the sugar having a critical relative humidity of 85% or more at 25°C is 90% by mass or more, and particularly preferably 100% by mass in the total excipient.

The orally administrable pharmaceutical composition of the present invention further contains various additives generally used, to the extent that the effects of the present invention are not prevented. The additive is selected from one generally used, namely excipients other than the aforementioned sugar having a critical relative humidity of 85% or more at 25°C, binders, lubricants, flavoring agents, colorants, and taste-masking agents.

The binder is selected from hydroxypropyl cellulose, hypromellose, and polyvinyl alcohol. The lubricants is selected from hydrogenated oils, sucrose fatty acid esters, and stearic acid. Examples of the colorant include food yellow No. 5, food blue No. 2, food lake, ferric oxide, yellow ferric oxide, and titanium oxide. Examples of the flavoring agent include various orange and lemon perfumes. Examples of the taste-masking agent include I-menthol, camphor, and mint. These may be used singly or in combination of two or more.

The content of the binder herein is from 0.001 to 5% by mass and preferably from 0.01 to 3% by mass in the total composition. The content of the lubricant is from 0.001 to 3% by mass and preferably from 0.01 to 2% by mass in the total composition.

The form of the orally administrable pharmaceutical composition of the present invention is selected from granules, compression-molded products (for example, uncoated tablets), and mixtures.

Also, the orally administrable pharmaceutical composition of the present invention, from a viewpoint of securing storage stability of the active ingredients, is preferably substantially free of metal salts, such as alkali metal salts and alkaline earth metal salts. "is substantially free" herein refers to from 0 to 0.1 parts by mass, preferably from 0 to 0.05 parts by mass, more preferably from 0 to 0.01 parts by mass, and still more preferably 0 parts by mass, based on 1 part by mass of FTD.

Although the orally administrable pharmaceutical composition of the present invention may be used as it is as a pharmaceutical formulation, the formulation can be further coated on its surface to be an orally administrable pharmaceutical formulation which is stable and easily ingested. Coating herein includes film coating and sugar coating. Examples of a coating base include hypromellose, ethyl cellulose, hydroxypropyl cellulose, polyvinyl alcohol, and sucrose. It should be noted that, in the case of coating an orally administrable pharmaceutical composition comprising FTD and TPI, the coating layer may contain the aforementioned additive having a critical relative humidity less than 85% or having no critical relative humidity to the extent that the stability of FTD and TPI is not substantially influenced. Also, in the case of coating an orally administrable pharmaceutical composition containing FTD and TPI, the coating layer may contain a small amount of plasticizers, colorants, flavoring agents, taste-masking agents, and lubricants to the extent that the stability of FTD and TPI is not substantially influenced. Examples of the plasticizer include polyethylene glycol. Examples of the colorant include food tar dyes, food tar dye lakes, ferric oxide, yellow ferric oxide, and titanium oxide. Examples of the flavoring agent include various orange and lemon perfumes. Examples of the taste-masking agent include l-menthol, camphor, and mint, which may be used singly or in combination of two or more. The total amount of the coating layer herein is preferably from 1 to 5% by mass and more preferably from 2 to 4% by mass in the total formulation.

Examples of the orally administrable pharmaceutical formulation of the present invention include tablets, granules, powders, and fine granules. Examples of the tablets include chewable tablets, troches, drops, and compositions which quickly dissolve or disintegrate in the mouth cavity and can be ingested even without water, and also include effervescent tablets which are dissolved to be used at time of use. Examples of the granules, powders, and fine granules include dry syrups which are dissolved to be used at time of use, and also include powder particles which quickly dissolve in the mouth cavity and can be ingested without water.

The orally administrable pharmaceutical composition and pharmaceutical formulation of the present invention can be produced in accordance with the known method for producing orally administrable formulations. Examples of the granulation method include fluid bed granulation methods, stirring granulation methods, tumbling fluid bed granulation methods, extruding granulation methods, spray granulation methods, and crushing granulation methods, which can be used to produce granules or uncoated tablets. Also, from a viewpoint of the granulation principles, granulation methods are largely divided into the dry granulation method and the wet granulation method. From a viewpoint of the stability of FTD and TPI, the dry granulation method is preferred.

According to the present invention, adding the sugar can suppress increases in formation of related substances of FTD and TPI which are potentially formed when orally administrable pharmaceutical compositions and pharmaceutical formulations comprising FTD and TPI as active ingredients are produced. The corresponding related substances herein mean components other than FTD, TPI, and additives, and mainly refer to structurally related compounds of the corresponding two active ingredients. Specifically, the related substances are substances other than FTD, TPI, and additives which are detected when measured in accordance with Liquid Chromatography described in the Japanese Pharmacopoeia, General Tests, Physical tests, after the orally administrable pharmaceutical composition and pharmaceutical formulation of the present invention are stored under certain constant conditions.

### [Examples]

Although the present invention is described in more details hereinbelow referring to Examples, Comparative Examples, Reference Examples, and Test Examples, the present invention is not intended to be limited solely by these Examples.

### Example 1 (Reference)

In a mortar, 40 g of FTD and 18.84 g of TP were mixed. In a mortar, 16. g of this mixture and 8g of a lactose hydrate "Lactochem DOMO" (manufactured by DMV-Fontera Excipients GmbH &Co) were mixed to thereby obtain a mixture (see Table 1.) It should be noted that the proportion of the corresponding sugars in additives is 10% in this composition.

### Example 2 (Reference)

A mixture was obtained in accordance with the same method as in Example 1, except that sucrose "Granulated sugar EA" (manufactured by ENSUIKO Sugar Refining Co., Ltd.) was used instead of the lactose hydrate.

### Example 3 (Reference)

In a plastic bag, 105 g of FTD and 49.5 g of TPI were mixed. In a tablet crusher (manufactured by Konishi- Seisakusho Co., Ltd.), 6.0 g of this mixture and 24 g of a lactose hydrate "Lactochem DOMO" (manufactured by DMV- Fonterra Excipients GmbH &Co) were mixed. Purified water was further added to this mixture, which was granulated, and then dried in Mini Jet Oven (manufactured by TOYAMA SANGYO CO., LTD.) at 70°C for wt o hours to thereby obtain granules (see Table 2). tl should be noted that the proportion of the corresponding sugars in additives is 100% in this composition.

### Example 4

A granule was obtained in accordance with the same method as in Example 3, except that D-mannitol (manufactured by KYOWA HAKKO BIO CO., LTD.) was used instead of the lactose hydrate (see Table 2).

### Comparative Example 1

A mixture was obtained in accordance with the same method as in Example 1, except that crystalline cellulose "Ceolus" (manufactured by Asahi Kasei Corporation) was used instead of the lactose hydrate (see Table 1).

### Comparative Example 2

A granule was obtained in accordance with the same method as in Example 3, except that D-sorbitol (manufactured by Towa Chemical Industry Co., Ltd.) was used instead of the lactose hydrate (see Table 2).

### Comparative Example 3

A granule was obtained in accordance with the same method as in Example 3, except that xylitol (manufactured by Towa Chemical Industry Co., Ltd.) was used instead of the lactose hydrate (see Table 2).

### Reference Example 1

In a mortar, 40 g of FTD and 18.84 g of TPI were mixed to thereby obtain a mixture (see Table 1).

### Test Example 1

The critical relative humidity of additives at 25°C shown in Tables 1 and 2 was measured using a moisture sorption analyzer (DVS-1, Surface Measurement Systems Ltd.). The results are shown in Tables 1 and 2.

### Test Example 2

The mixtures obtained in Examples 1 and 2, Comparative Example 1, and Reference Example 1 were stored at 40°C/75% R.H. for a month, and then, the mass of the related substances formed was measured in accordance with Liquid Chromatography described in the Japanese Pharmacopoeia, General Tests, Physical tests. The results are shown in Table 1. It should be noted that peaks other than those of FTD, TPI, and additives are called related substance peaks and that the total mass of the related substances refers to the sum of the mass of the related substances calculated based on the area of the active ingredients from the area of the related substance peaks.

### Test Example 3

In accordance with the method described in Test Example 2, the granules obtained in Examples 3 and 4, and Comparative Examples 2 and 3 were stored at 40°C/75% R.H. for a week, and then, the mass of the formed related substances was measured in accordance with Liquid Chromatography described in the Japanese Pharmacopoeia, General Tests, Physical tests. The results are shown in Table 2.

**[Table 1]**

| | Unit: parts by mass | | | |
|---|---|---|---|---|
| | Example | | Comparative Example | Reference Example |
| | 1 | 2 | 1 | 1 |
| FTD | 10 | 10 | 10 | 10 |
| TPI | 4.71 | 4.71 | 4.71 | 4.71 |
| Lactose hydrate | 73.55 | - | - | - |
| Sucrose | - | 73.55 | - | - |
| Crystalline cellulose | - | - | 73.55 | - |
| Critical relative humidity (%, at 25°C) | 95 or more | 85 or more | Not applicable | - |
| Total mass of the related substances (%) | 0.19 | 0.36 | 1.64 | 0.15 |

**[Table 2]**

| | Unit: parts by mass | | | |
|---|---|---|---|---|
| | Example | | Comparative Example | |
| | 3 | 4 | 2 | 3 |
| FTD | 10 | 10 | 10 | 10 |
| TPI | 4.71 | 4.71 | 4.71 | 4.71 |
| Lactose hydrate | 58.84 | - | - | - |
| D-mannitol | - | 58.84 | - | - |
| D-sorbitol | - | - | 58.84 | - |
| Xylitol | - | - | - | 58.84 |
| Critical relative humidity (%, at 25°C) | 95 or more | 95 or more | 50-60 | 75-85 |
| Total mass of the related substances (%) | 0.08 | 0.00 | 0.81 | 0.63 |

As clearly seen from Table 1, the total mass of the related substances of Examples 1 and 2 in which a sugar having a critical relative humidity of 85% or more at 25°C was used as the excipient showed virtually no difference compared to Reference Example 1, and was very stable compared to Comparative Example 1. Also, from Table 2, the total mass of the related substances of Examples 3 and 4 in which a sugar having a critical relative humidity of 85% or more at 25°C was used as the excipient was clearly less than that of Comparative Examples 2 and 3 in which a sugar having a critical relative humidity of less than 85% at 25°C was used as the excipient, and was very stable.

### Example 4 (Reference)

From the above-described result, it was found that FTD and TPI-containing formulations having high stability even under severe conditions such as 40°C/75% R.H. can be obtained by using a sugar having a critical relative humidity of 85% or more at 25°C as the excipient. Since formation of related substances is suppressed, it is possible to provide patients and medical staffs with formulations of higher quality.

### Example 5 (Reference)

In a plastic bag, 400 g of FTD, 188.4 g of TPI, 1511.6 g of a lactose hydrate, 300 g of carmellose "NS-300" (manufactured by GOTOKU CHEMICAL COMPANY LTD), and 40 g of stearic acid were mixed. This mixture was tableted with a rotary tableting machine into tablets having a diameter of 15 m and a mass of 800 mg. Then, the tablets were crushed with a crusher to thereby obtain a granule. To 122 parts of this granule, 1 part of stearic acid was further added and mixed in a plastic bag. Uncoated tablets having a diameter of 7 m and a mass of 123 mg were obtained by use of a rotary tableting machine (see Table 3).

### Example 6 (Reference)

In a mortar, 1 g of a mixture of 1 part of FTD and 0.471 parts of TPI, 6 g of a lactose hydrate, and 1 g of carmellose were mixed. From this mixture, uncoated tablets having a mass of 235.36 mg were obtained by use of a hydraulic press (see Table 3).

### Example 7

In a plastic bag, 1200 g of FTD, 565.2 g of TPI, 7258.8 g of a lactose hydrate, 480 g of partly pregelatinized starch "PCS(PC-10)" (manufactured by Asahi Kasei Chemicals Corporation), and 96 g of stearic acid were mixed. From this mixture, uncoated tables having a diameter of 7 mm and a mass of 120 mg were obtained by use of a rotary tableting machine (see Table 3).

### Example 8

In accordance with the method described in Example 7, 100 g of FTD, 47.1 g of TPI, 371.9 g of a lactose hydrate, 100 g of partly pregelatinized starch, and 6 g of stearic acid were mixed in a plastic bag. From this mixture, uncoated tablets having a diameter of 7 mm and a mass of 125 mg were obtained by use of a rotary tableting machine (see Table 4).

### Example 9

In accordance with the method described in Example 7, 100 g of FTD, 47.1 g of TPI, 371.9 g of a lactose hydrate, 25 g of partly pregelatinized starch, and 6 g of stearic acid were mixed in a plastic bag. From this mixture, uncoated tablets having a diameter of 7 mm and a mass of 110 mg were obtained by use of a rotary tableting machine (see Table 4).

### Example 10

In according with the method described in Example 7, 100 g of FTD, 47.1 g of TPI, 371.9 g of a lactose hydrate, 50 g of partly pregelatinized starch, and 6 g of stearic acid were mixed in a plastic bag. From this mixture, uncoated tablets having a diameter of 7 mm and a mass of 115 mg were obtained by use of a rotary tableting machine (see Table 4).

### Example 11

In accordance with the method described in Example 7, 100 g of FTD, 47.1 g of TPI, 521.9 g of a lactose hydrate, 75 g of partly pregelatinized starch, and 6 g of stearic acid were mixed in a plastic bag. From this mixture, uncoated tablets having a diameter of 7 mm and a mass of 150 mg were obtained by use of a rotary tableting machine (see Table 4).

### Example 12

In accordance with the method described in Example 7, 100 g of FTD, 47.1 g of TPI, 671.9 g of a lactose hydrate, 75 g of partly pregelatinized starch, and 6 g of stearic acid were mixed in a plastic bag. From this mixture, uncoated tablets having a diameter of 7m and amas of 150 mg were obtained by use of a rotary tableting machine (see Table 4).

**[Table 3]**

| Unit: parts by mass | | | |
|---|---|---|---|
| | Example | | |
| | 5 | 6 | 7 |
| FTD | 1 | 1 | 1 |
| TPI | 0.47 | 0.47 | 0.47 |
| Lactose hydrate | 3.78 | 8.83 | 6.05 |
| Carmellose | 0.75 | 1.47 | - |
| Partly pregelatinized starch | - | | 0.4 |
| Stearic acid | 0.15 | - | 0.08 |
| Total | 6.15 | 11.77 | 8 |

**[Table 4]**

| | Unit: parts by mass | | | | |
|---|---|---|---|---|---|
| | Example | | | | |
| | 8 | 9 | 10 | 11 | 12 |
| FTD | 1 | 1 | 1 | 1 | 1 |
| TPI | 0.471 | 0.471 | 0.471 | 0.471 | 0.471 |
| Lactose hydrate | 3.719 | 3.719 | 3.719 | 5.219 | 6.719 |
| Partly pregelatinized starch | 1 | 0.25 | 0.5 | 0.75 | 0.75 |
| Stearic acid | 0.06 | 0.06 | 0.06 | 0.06 | 0.06 |
| Total | 6.25 | 5.5 | 5.75 | 7.5 | 9 |

### Example 13 (Reference)

In a mortar, 1g of FTD, 0.471 g of TPI, 3.779 g of a lactose hydrate, and 0.15 g of stearic acid were mixed. From this mixture, uncoated tablets having a mas of 108 mg were obtained by use of a hydraulic press (see Table 5).

### Example 14 (Reference)

In a plastic bag, 1 g of FTD, 0.471 g of TPI, 3.779 g of a lactose hydrate, 0.75 g of carmellose as a disintegrating agent, and 0.15 g of stearic acid were mixed. From this mixture, uncoated tablets having a mass of 123 mg were obtained by use of a hydraulic press (see
Table 5).

### Comparative Example 4

In accordance with the method described in Example 14, 0.75 g of carmellose calcium "E.C.G-505" (manufactured by GOTOKU CHEMICAL COMPANY LTD.) was used as a disintegrating agent instead of carmellose to thereby obtain uncoated tablets having a mass of 123 mg (see Table 5).

### Comparative Example 5

In accordance with the method described in Example 14, 0.75 g of croscarmellose sodium "Ac-Di-Sol" (manufactured by Asahi Kasei Corporation) was used as a disintegrating agent instead of carmellose to thereby obtain uncoated tablets having a mass of 123 mg (see Table 5).

### Test Example 4

In accordance with the method described in Test Example 2, tablets obtained in Examples 13 and 14 and Comparative Examples 4 and 5 were stored at 40°C/75% R.H. in open conditions for one month, and then, the total mass of the related substances was measured (see Table 5).

As the result, even if carmellose, which is a disintegrating agent having no critical relative humidity, was contained, it was found that the disintegrability as orally administrable tablets was sufficiently secured, noticeable increases in related substances were not observed, and the storage stability was secured. In contrast, if carmellose calcium or croscarmellose sodium was contained as a disintegrating agent, the mass of related substances was noticeably increased, and the storage stability was not secured.

**[Table 5]**

| | Unit: parts by mass | | | |
|---|---|---|---|---|
| | Example | | Comparative Example | |
| | 13 | 14 | 4 | 5 |
| FTD | 1 | 1 | 1 | 1 |
| TPI | 0.471 | 0.471 | 0.471 | 0.471 |
| Lactose hydrate | 3.779 | 3.779 | 3.779 | 3.779 |
| Carmellose | - | 0.75 | - | - |
| Carmellose calcium | - | - | 0.75 | - |
| Croscarmellose sodium | - | - | - | 0.75 |
| Stearic acid | 0.15 | 0.15 | 0.15 | 0.15 |
| Total | 5.4 | 6.15 | 6.15 | 6.15 |
| Total mass of the related substances (%) | 0.286 | 0.404 | 1.194 | 2.529 |

### Example 15 (Reference)

In accordance with the method described in Example 7, 50 g of FTD, 23.55 g of TPI, 226.45 g of a lactose hydrate, and 3 g of stearic acid were mixed in aplastic bag. From this mixture, uncoated tablets having a mas of 121.2 mg were obtained by use of a rotary tableting machine (se Tabel 6.)

### Example 16

In accordance with the method described in Example 7, 50 g of FTD, 23.55 g of TPI, 211.45 g of a lactose hydrate, 15 g of a disintegrating agent (any of corn starch "corn starch W" (manufactured by NIHON SHOKUHIN KAKO CO., LTD.) (Reference), partly pregelatinized starch (Invention), or low-substituted hydroxypropyl cellulose (Reference)), and 3 g of stearic acid were mixed in a plastic bag. From this mixture, uncoated tablets having amas of 121.2 mg were obtained by use of a rotary tableting machine (see Table 6).

### Example 17

In accordance with the method described in Example 16, 50 g of FTD, 23.55 g of TPI, 196.45 g of a lactose hydrate, 30 g of a disintegrating agent (any of corn starch (Reference), partly pregelatinized starch (Invention) or low-substituted hydroxypropyl cellulose (Reference)), and 3 g of stearic acid were mixed in a plastic bag. From this mixture, uncoated tablets having a mass of 121.2 mg were obtained by use of a rotary tableting machine (see Table 6).

### Test Example 5

In accordance with the method described in Test Example 2, tablets obtained in Examples 15, 16, and 17 were stored at 40°C/75% R.H. in open conditions for two weeks, and then, the total mass of the related substances was measured (see Table 6).

As the result, noticeable increases in related substances were not observed in any of the disintegrating agents and amounts thereof.

**[Table 6]**

| | Unit: parts by mass | | | | | | |
|---|---|---|---|---|---|---|---|
| | Example | | | | | | |
| | 15 | 16 | | | 17 | | |
| FTD | 1 | 1 | 1 | 1 | 1 | 1 | 1 |
| TPI | 0.471 | 0.471 | 0.471 | 0.471 | 0.471 | 0.471 | 0.471 |
| Lactose hydrate | 4.529 | 4.229 | 4.229 | 4.229 | 3.929 | 3.929 | 3.929 |
| Com starch | - | 0.3 | - | - | 0.6 | - | - |
| Partly pregelatinized starch | - | - | 0.3 | - | - | 0.6 | - |
| Low-substituted hydroxypropyl cellulose | - | - | - | 0.3 | - | - | 0.6 |
| Stearic acid | 0.06 | 0.06 | 0.06 | 0.06 | 0.06 | 0.06 | 0.06 |
| Total | 6.06 | 6.06 | 6.06 | 6.06 | 6.06 | 6.06 | 6.06 |
| Total mass of the related substances (%) | 0.188 | 0.2 | 0.266 | 0.332 | 0.282 | 0.334 | 0.391 |

## Claims

1. An orally administrable pharmaceutical composition consisting essentially of
(a) α,α,α-trifluorothymidine and 5-chloro-6-(2-iminopyrrolidine-1-yl) methyl-2,4 (1H,3H)-pyrimidine dione hydrochloride as active ingredients at a molar ratio of 1:0.5,
(b) a sugar having a critical relative humidity of 85% or more at 25 °C as an excipient, wherein the sugar having a critical relative humidity of 85% or more at 25°C is one or more selected from lactose, sucrose, mannitol, and erythritol,
(c) a disintegrating agent which is partly pregelatinized starch, **and**
(d) **additives selected from** excipients other than the sugar having a critical relative humidity of 85% or more at 25 °C,
0.001 to 5% by mass in the total composition of a binder selected from hydroxypropyl cellulose, hypromellose, and polyvinyl alcohol,
0.001 to 3% by mass in the total composition of a lubricant selected from hydrogenated oils, sucrose fatty acid esters, and stearic acid, flavoring agents,
colorants, and
taste-masking agents,
wherein the content of the sugar having a critical relative humidity of 85% or more at 25°C is 3.6 parts by mass or more based on 1 part by mass of α,α,α-trifluorothymidine,
wherein the proportion of the sugar having a critical relative humidity of 85% or more at 25 °C is 90% by mass or more in the total excipient,
wherein the content of the disintegrating agent is from 3 to 7% by mass in the total amount of the pharmaceutical composition, and
wherein the pharmaceutical composition is in a formulation form of a granule, a compression-molded product, or a mixture.

2. An orally administrable pharmaceutical formulation comprising the orally administrable composition according to claim 1, wherein the composition is coated.

## Patentansprüche

1. Oral verabreichbare pharmazeutische Zusammensetzung, im Wesentlichen bestehend aus
(a) α,α,α-Trifluorthymidin und 5-Chlor-6-(2-iminopyrrolidin-1-yl)methyl-2,4(1H,3H)-pyrimidindion-hydrochlorid als Wirkstoffe in einem Molverhältnis von 1:0,5,
(b) einem Zucker, der eine kritische relative Feuchte von 85 % oder mehr bei 25°C aufweist, als Hilfsstoff, wobei der Zucker, der eine kritische relative Feuchte von 85 % oder mehr bei 25°C aufweist, einer oder mehrere ist, ausgewählt aus Lactose, Saccharose, Mannitol und Erythritol,
(c) teilweise vorgelatinierte Stärke als Sprengmittel, und
(d) Zusatzstoffe, ausgewählt aus anderen Hilfsstoffen als Zucker mit einer kritischen relativen Luftfeuchtigkeit von 85 % oder mehr bei 25 °C,
0,001 bis 5 Massenprozent eines Bindemittels, ausgewählt aus Hydroxypropylcellulose, Hypromellose und Polyvinylalkohol,
0,001 bis 3 Massenprozent eines Schmiermittels, ausgewählt aus gehärteten Ölen, Saccharosefettsäureestern und Stearinsäure,
Aromastoffen,
Farbstoffen und
Geschmacksmaskierungsmitteln,
wobei der Gehalt des Zuckers mit einer kritischen relativen Luftfeuchtigkeit von 85 % oder mehr bei 25 °C 3,6 Massenteile oder mehr, bezogen auf 1 Massenteil α,α,α-Trifluorthymidin, beträgt,
wobei der Anteil des Zuckers mit einer kritischen relativen Luftfeuchtigkeit von 85 % oder mehr bei 25 °C 90 Massenprozent oder mehr am gesamten Hilfsstoff beträgt,
wobei der Gehalt Der Anteil des Sprengmittels 3 bis 7 Massenprozent der Gesamtmenge der pharmazeutischen Zusammensetzung beträgt, und
wobei die pharmazeutische Zusammensetzung in Form eines Granulats, eines formgepressten Produkts oder einer Mischung vorliegt.

2. Oral verabreichbare pharmazeutische Formulierung, umfassend die oral verabreichbare Zusammensetzung nach Anspruch 1, wobei die Zusammensetzung beschichtet ist.

## Revendications

1. Composition pharmaceutique pouvant être administrée par voie orale consistant essentiellement en :
(a) l'α,α,α-trifluorothymidine et le chlorhydrate de 5-chloro-6-(2-iminopyrrolidin-1-yl)méthyl-2,4(1H,3H)pyrimidinedione à titre d'agents actifs à un rapport molaire de 1/0,5,
(b) un sucre présentant une humidité relative critique de 85 % ou plus à 25 °C à titre d'excipient, dans laquelle le sucre présentant une humidité relative critique de 85 % ou plus à 25 °C est un ou plusieurs sélectionnés parmi le lactose, le saccharose, le mannitol, et l'érythritol,
(c) un agent de délitement qui est un amidon partiellement prégélatinisé, et
(d) des additifs sélectionnés parmi des excipients autres que le sucre présentant une humidité relative critique de 85 % ou plus à 25 °C,
0,001 à 5 % en masse de la composition totale d'un liant sélectionné parmi une hydroxypropylcellulose, une hypromellose et un poly(alcool vinylique),
0,001 à 3 % en masse de la composition totale d'un lubrifiant sélectionné parmi les huiles hydrogénées, les esters de saccharose d'acide gras, et l'acide stéarique,
les agents aromatisants,
les colorants, et
les agents de masquage de goût,
dans laquelle la teneur en sucre présentant une humidité relative critique de 85 % ou plus à 25 °C est de 3,6 parties en masse ou plus sur la base de 1 partie en masse de l'α,α,α-trifluorothymidine,
dans laquelle la proportion du sucre présentant une humidité relative critique de 85 % ou plus à 25 °C est de 90 % en masse ou plus dans l'excipient total,
dans laquelle la teneur en agent de délitement est de 3 à 7 % en masse dans la quantité totale de la composition pharmaceutique, et
dans laquelle la composition pharmaceutique est sous une forme de formulation d'un granule, d'un produit moulé par compression, ou d'un mélange.

2. Formulation pharmaceutique pouvant être administrée par voie orale comprenant la composition pouvant être administrée par voie orale selon la revendication 1, dans laquelle la composition est enrobée.
